Europäisches Patentamt

(19) European Patent Office

Office européen des brevets

(11) Publication number: **0 070 186**

**B1**

(12) **EUROPEAN PATENT SPECIFICATION**

(45) Date of publication of patent specification: **10.07.85**

(21) Application number: **82303666.0**

(22) Date of filing: **13.07.82**

(51) Int. Cl.[4]: **C 07 K 7/10,** A 61 K 37/02,
A 61 K 39/00

(54) Biologically active peptides.

(30) Priority: **15.07.81 GB 8121699**
**26.11.81 GB 8135738**
**07.04.82 GB 8210297**

(43) Date of publication of application:
**19.01.83 Bulletin 83/03**

(45) Publication of the grant of the patent:
**10.07.85 Bulletin 85/28**

(84) Designated Contracting States:
**CH DE FR GB IT LI NL SE**

(56) References cited:
NATURE, vol. 295, 28th January 1982, pages
345-347
CHEMICAL ABSTRACTS, vol. 96, 1982, page
116, no. 46807d, Columbus, Ohio, USA
CHEMICAL ABSTRACTS, vol. 96, 1982, page
369, no. 49477g, Columbus, Ohio, USA
CHEMICAL ABSTRACTS, vol. 83, no. 9, 1975,
page 409, no. 75785g, Columbus, Ohio, USA
CHEMICAL ABSTRACTS, vol. 95, no. 25, 1981,
page 182, no. 216568a, Columbus, Ohio, USA

(73) Proprietor: **CELLTECH LIMITED**
**244-250 Bath Road**
**Slough Berkshire SL1 4DY (GB)**

(72) Inventor: **Craig, Roger Kingdon The Courtauld**
**Institute**
**of Biochemistry The Middlesex Hospital School**
**London W1P 7PN (GB)**
Inventor: **MacIntyre, Iain**
**The Royal Postgraduate Medical School**
**Hammersmith Hospital London W12 0HS (GB)**

(74) Representative: **Votier, Sidney David et al**
**CARPMAELS & RANSFORD 43, Bloomsbury**
**Square**
**London WC1A 2RA (GB)**

Courier Press, Leamington Spa, England.

## Description

This invention relates to biologically active peptides and in particular to biologically active flanking peptides of human calcitonin precursor. Calcitonin is a relatively small peptide hormone (mol. wt. 23500), having as its major role the protection of the skeleton during periods of calcium stress such as growth, pregnancy and lactation. It is secreted by C-cells which are within the thyroid in mammals, but may be mainly in the ultimobranchial body and lung of more primitive vertebrates. The hormone is also concentrated in the hypothalamus of several vertebrate species, including man, as well as in the primitive brain of the chordate Ciona intestinalis. Calcitonin has been used therapeutically for treatment in abnormal situations of elevated bone turnover such as Paget's disease. Chemically synthesised non-human calcitonins, such as salmon calcitonin, are widely used for such therapy.

In our accompanying Application of even date, entitled "Human Calcitonin Precursor Polyprotein Structural Gene", the disclosure of which is incorporated by reference, we have described the production of human calcitonin by recombinant DNA techniques. Such a route to the production of calcitonin avoids complex and costly peptide chemistry and can provide a product which is identical with the human hormone and thus advantageously free from undesirable immune response problems, for instance, when administered over prolonged periods. Studies of mRNA directed cell-free protein synthesis provide evidence that calcitonin is synthesised as a high molecular weight precursor protein. Subsequent application of recominant DNA techniques has resulted in the cloning of the structural gene of this precursor protein. Nucleotide sequencing of the cloned cDNA has revealed that human calcitonin resides towards the carboxyl terminal of the precursor polyprotein, flanked at amino and carboxyl termini by previously unknown cryptic peptide sequences.

It has now been found that these flanking sequences provide peptides having useful biological activity and diagnostic properties.

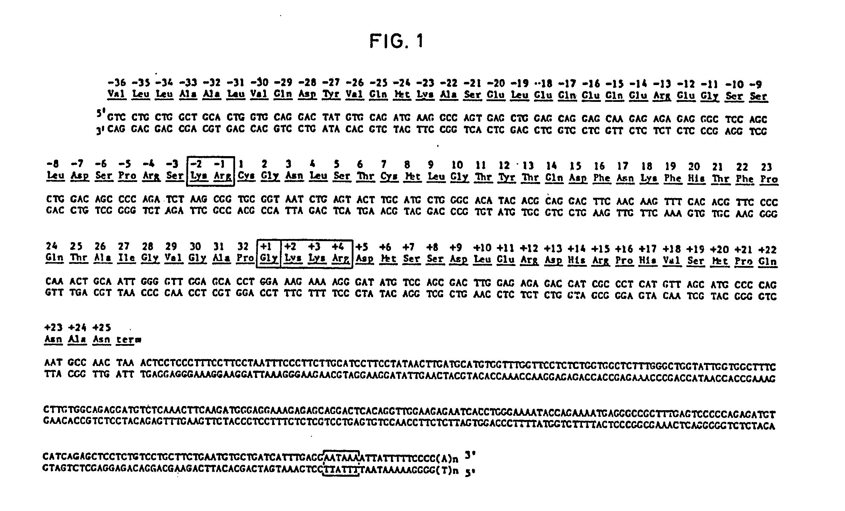

The flanking peptide sequences of human calcitonin are those sequences shown in whole for the C terminal and in part for the N terminal, in Figure 1 herewith, which is a diagram of the nucleotide sequence of human calcitonin precursor polyprotein. The N-terminal sequence is the −36 to −1 sequence and the C-terminal sequence is +1 to +25. Calcitonin is 1 to 32. Boxed sections on each side of the calcitonin sequence (−2, −1 and +1 to +4) indicate amino acids which are probable sites of proteolytic processing and may not be present in processed secreted peptides.

The present invention provides a polypeptide having the carboxyl terminal peptide sequence, i.e. the +5 to +25 sequence Asp - Met - Ser - Ser - Asp - Leu - Glu - Arg - Asp - His - Arg - Pro - His - Val - Ser - Met - Pro - Gln - Asn - Ala - Asn, hereinafter referred to as PDN-21. The designation of this peptide follows the terminology of Tatemoto and Mutt, Proc. Natl. Acad. Sci. U.S.A. 78, 6603—6607 (1981).

The peptides of the invention are typically in substantially purified form.

It has been found that PDN-21 is highly biologically active. Thus, it is as potent as thyrotropin releasing hormone (TRH) in releasing prolactin. Further it approaches the potency of calcitonin in lowering plasma calcium. For example, it has been found that intravenous injection of PDN-21 into rats is accompanied by a lowering of the plasma calcium concentration as determined by a "calcitonin" bioassay procedure. And since immunological evidence suggest that PDN-21 circulates in vivo, it represents a new hormonal cleavage product from the human calcitonin precursor with a physiological role as a neuropeptide and in calcium regulation.

Furthermore, it has been found that injection of PDN-21 together with calcitonin appears to give rise to an advantageous synergistic effect, lowering plasma calcium to a greater extent than would be expected from the sum of the separate effects of PDN-21 and calcitonin. It is believed that other peptides according to the invention will exhibit similar neuropeptide and calcium regulating activity and corresponding synergistic activity to that of PDN-21.

The invention also includes compositions comprising the peptides of the invention, especially pharmaceutically acceptable, e.g. injectable, compositions comprising peptide in combination with pharmaceutically acceptable diluent, excipient, preservative etc. In a particularly preferred embodiment the invention includes synergistic compositions comprising a peptide of the invention in combination with calcitonin.

Peptides according to the invention may be prepared by any appropriate method. The peptides may be prepared by partial or complete chemical synthesis using methods well known in the art, for instance by solid phase peptide synthesis. Also the peptides of the invention may be prepared using recombinant DNA techniques.

The peptides of the invention may be used therapeutically to protect bone against direct attack in a similar fashion to calcitonin and to restrain osteoclastic breakdown. It is believed that the peptides may have therapeutic activity alone or in combination with calcitonin in bone disorders where resorption is increased e.g. Paget's disease or osteoporosis. The peptides may also be used in their role as neuropeptides, for instance PDN-21 acting as a prolactin releasing factor may prove useful in the induction or prolongation of lactation in females or as a contraceptive in males through its role in the release of pituitary prolactin.

The peptides of the invention may also be used in connection with the diagnosis of disorders which are associated with elevated serum calcitonin levels. For instance, the peptides may be used for the production of polyclonal or monoclonal antibodies for use in immunoassay detection and determination of calcitonin precursor or its flanking peptide sequences, and the invention therefore includes antibodies to the peptides e.g. anti PDN-21. Such antibodies may be used for diagnosis of thyroid disorders such as medullary carcinoma of the thyroid, and also for diagnosis of disorders in which there is ectopic secretion of calcitonin precursor or its flanking peptide sequences e.g. oat cell carcinoma of the lung, carcinoid tumours, endocrine tumours of the gut and pancreas and other cases of malignancy.

In the Examples which follow, for the purpose of illustration and not of limitation, the human C-terminal flanking cryptic peptide PDN-21 and a portion of the amino-terminal peptide PYE-14 were synthesised by solid phase methodology, and the identity and homogeneity of the products established by amino acid analysis, high voltage electrophoresis and reverse phase high pressure liquid chromatography. These were used, as described below, to obtain evidence of biological activity and to raise the antisera required to establish its presence in human tissues and sera.

Example 1

A "calcitonin" bioassay was carried out using groups of female Wistar rats of mean body weight 50 g (range 47—53 g). Six rats were assigned to a control group (Group 1) injected with buffer alone, and the other groups of rats (Groups 2—7) were injected intravenously with various calcitonin, PDN-21 and PDN-21/calcitonin combination treatments using the same volumes of buffer as used for the controls. The group injected with 1 μg/rat of PDN-21 (Group 4) consisted of 7 rats, whereas the other injected groups consisted of 5 rats each. Thirty minutes after injection the rats in all groups were sacrificed and the blood of each rat was sampled from the aorta and its calcium level determined by methods known in the art.

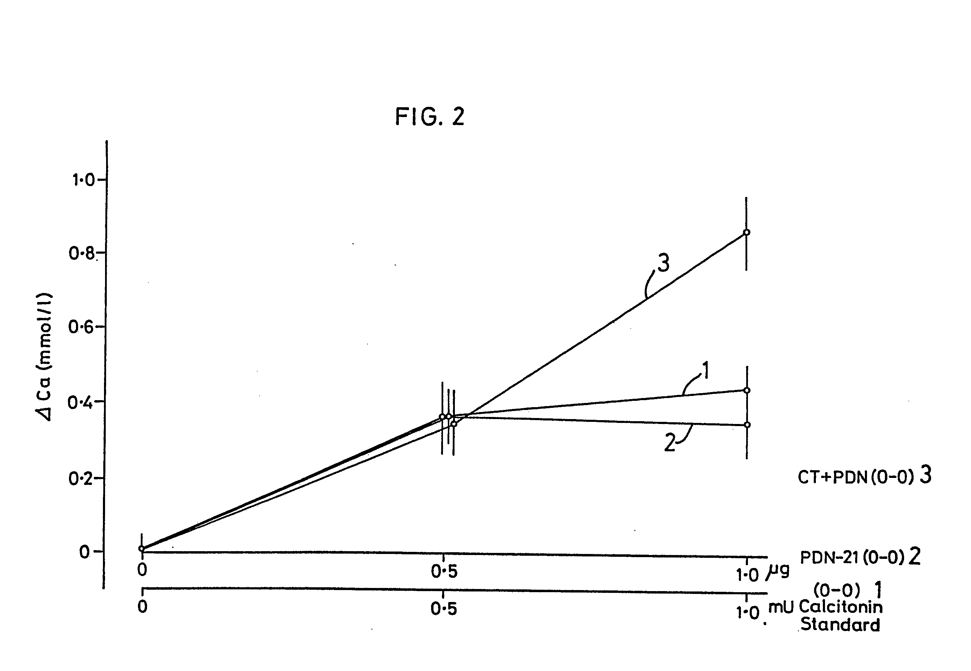

The results obtained are given in Table I below and in graphical form in Figure 2 which is a plot of the decrease in serum calcium concentration against the amount of injected composition. The doses of calcitonin administered (curve 1) to each rat are given in terms of mU (MRC milliunits) which is a universal standard known in the art; whereas the doses of PDN-21 (curve 2) used are given in terms of μg of peptide/rat. The results show that, similar to calcitonin, PDN-21 injection is followed by lowering of plasma calcium. Furthermore, injection with a combined calcitonin/PDN-21 treatment (curve 3), at least at the higher level tested (calcitonin 1mU+PDN-21 1 μg/rat) appears to give a synergistic effect.

TABLE I
Bioassay
Plasma calcium levels (mmol/l)

Group 1—controls (buffer only)
2.58
2.58
2.59
2.54
2.50
2.68    m̄=2.578

Group 2—calcitonin standard 1 mU/rat.
2.07
2.28
2.05
2.25
2.05    m̄=2.140    p<0.001 compared to control

Group 3—calcitonin standard 0.5 mU/rat
2.32
2.30
2.23
2.28
1.93    m̄=2.212    p<0.001 compared to control

Group 4—PDN21 1 μg/rat
1.97
2.25
2.53
2.30
2.37
2.40
1.77    m̄=2.227    p<0.01 compared to control

Group 5—PDN21 0.5 μg/rat
1.78
2.17
2.60
2.74
1.75    m̄=2.203    p<0.1 (NS) compared to control

Group 6—calcitonin 1 mU+PDN 1 μg/rat
2.17
1.77
1.73
1.55
1.37    m̄—1.716    p<0.001 compared to control
p<0.02 compared to group 2

Group 7—calcitonin 0.5 mU+PDN 0.5 μg/rat
2.37
2.37
2.29
2.03
2.12    m̄=2.236    p<compared to control
NS compared to group 3

## Example 2
## The effect of PDN-21 on plasma calcium

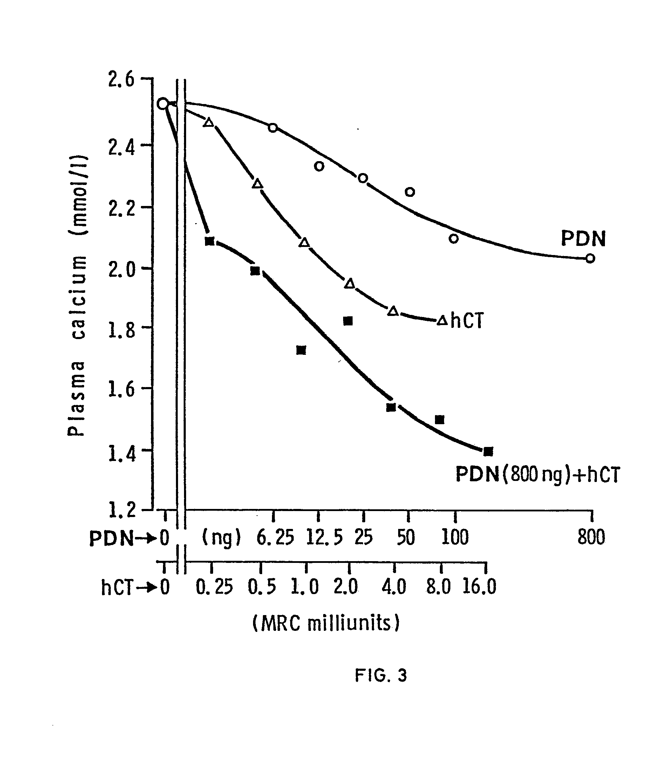

After preliminary experiments had shown that the time courses of the effects of PDN 21 and human calcitonin (hCT) on plasma calcium were similar, peptide in acetate buffer was injected into the tail vein of female Wistar rats (mean wt 45 g, range 43—48, Oxfordshire Laboratory Animal Colonies (1976) Ltd.) and blood was taken from the aorta after 30 min in a randomized bioassay (MacIntyre, I. Galante, L. S. & Hillyard, C. J. in *Handbuch der inneren Medizin* VI/IA (eds) Kuhlencordt, F. & Bartelheimer, H. 623—634 (Springer-Verlag, Heidelberg, 1980)). Plasma calcium was measured colormetrically by the method of (Gindler, E. M. & King, J. D. *Amer. J.Clin. Path.* 58, 376—382 (1972)), and the values confirmed by atomic absorption spectrophotometry. The results are shown graphically in Figure 3. Each point is the mean from three rats ○ PDN-21; △ hCT (WHO 1st International Reference Preparation of Calcitonin Hormone for Bioassay, 70/234 supplied by the National Institute for Biological Standards and Control); □ hCT+PDN-21 (800 ng PDN 21 was injected with each dose of calcitonin). Analysis of variance showed significant ($p < 0.001$) dose related plasma calcium differences among the three treatment groups. Smoothed curves were fitted to the data by eye. Probit transformation allowed calculation of the doses giving 50% response (ED50). These were 0.71 MRC milliunits and 24 ng for hCT and PDN-21 respectively. Potencies were calculated in terms of hCT over the dose ranges where the log dose response is linear, yielding values of 0.017 milliunits hCT equivalent/ng PDN-21 and 5.11 milliunits hCT equivalent/milliunit hCT in the hCT+PDN-21 group. The "potencies" reflect only the relative effects of these materials on calcium levels over the linear part of the log dose response range. Three previous unrandomized experiments yielded essentially similar results.

## Example 3

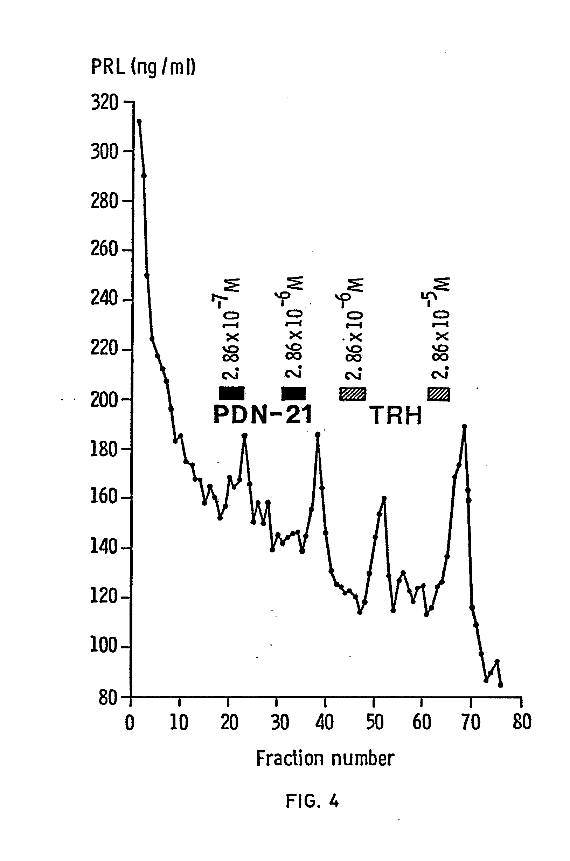

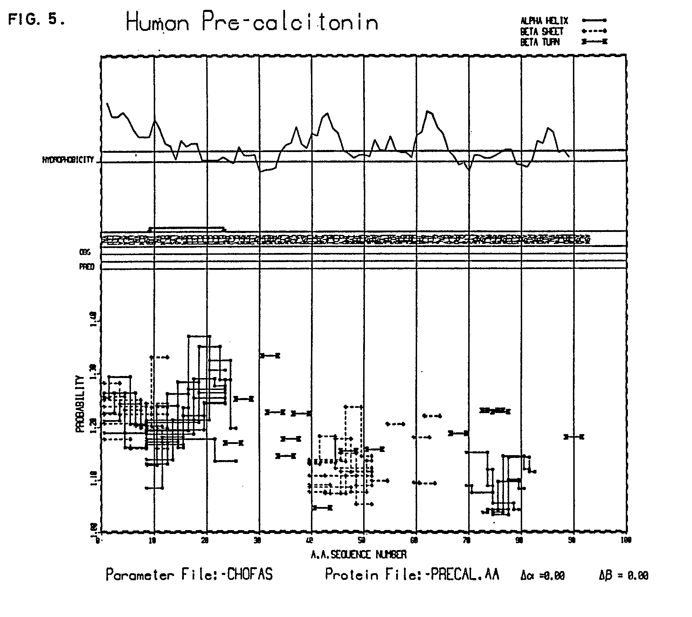

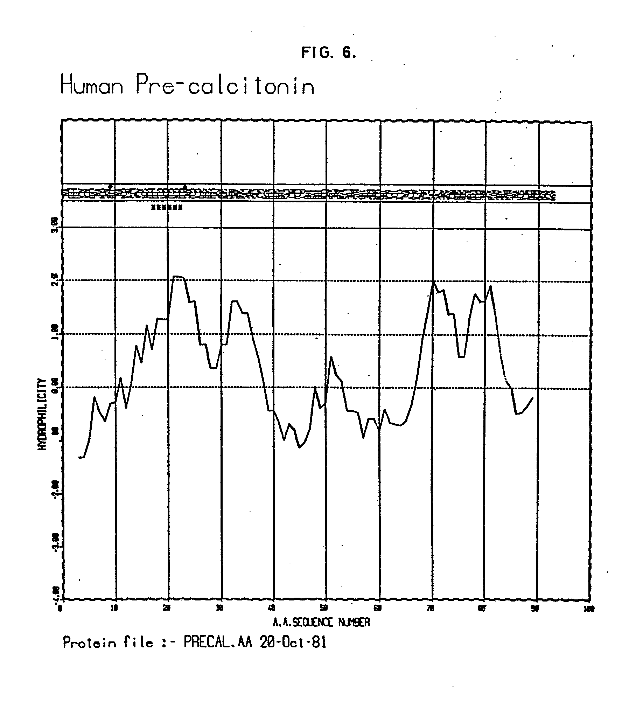

Figure 4 shows the concentration of prolactin in consecutive 1 ml fractions collected from an anterior pituitary cell column before, during and after perfusion with 10 min pulses (shown by bars) of two concentrations of PDN 21 ($2.86 \times 10^{-7}$ M and $2.86 \times 10^{-6}$ M) and TRH ($2.86 \times 10^{-6}$ M and $2.86 \times 10^{-5}$ M). The PDN-21, like TRH, stimulated prolactin release in a concentration-dependent manner. In terms of the total amount of prolactin released in excess of basal secretion, the equimolar doses ($2.86 \times 10^{-6}$ M) of PDN-21 and TRH released similar amounts of prolactin. PDN-21 released 52.1% and TRH released 50.3% relative to the amount of prolactin released by TRH ($2.86 \times 10^{-5}$ M). The lower dose of PDN-21 ($2.86 \times 10^{-7}$ M) released 35.6% relative to TRH ($2.86 \times 10^{-5}$ M).

The pituitary cell column was prepared according to Speight & Fink *J. Endocr.* 89, 129—134 (1981). Briefly, cells from the anterior pituitary glands of four male Wistar rats (200—250 g body weight; supplied by Bush Animal Breeding Station, Edinburgh) were dissociated according to the method of Hopkins & Farquhar *J.Cell Biol.* 59, 276—303 (1973). The cells obtained were mixed with Bio Gel P2 (200—400 mesh, Bio-Rad Laboratories, Richmond, California, U.S.A.) and a single cell column for perfusion was prepared as described by Lowry *J. Endocr.* 62, 163—164 (1974) and Gillies & Lowry *Endocrinology* 103, 521—527 (1978). The column was immersed in a water bath at 37°C, and perfused with gassed (95% $O_2$:5% $CO_2$) Dublecco's modified Eagle medium—1% BSA (DMEM-BSA) at a flow rate of 0.5 ml/min. After washing the column for 3 hours the effects of PDN 21 and TRH were examined. Each pulse of test hormone was separated by a 20 min period of DMEM-BSA alone.

The graphical results show that PDN-21 stimulated prolactin release in a concentration dependent manner and was, on a molar basis, about as potent as TRH.

## Example 4

Comparison of the effectiveness of human calcitonin in inhibiting cell-mediated $^{45}$Ca release from prelabelled mouse calvariae in vitro.

The method for studying the response of mouse bones to calcitonin preparations has been described in detail by Reynolds, J. J. Minkin, C. & Parsons, J.A. *Calcif. Tiss. Res.* 4, 350—358 (1970). Cell-mediated release of $^{45}$Ca into culture medium was calculated by subtracting the amount of exchangeable $^{45}$Ca released into medium by dead explants of 6 day old prelabelled mice of the same litter from that of the living treated explant (see Reynolds, J.J. in *Organ Culture in Biomedical Research* (eds) Balls, M. & Monnickendam, M. 355—366 (Cambridge University Press, Cambridge, 1976). The results are shown in Table II below. The number of treated explants is shown in parentheses; the data were obtained after treatment of explants for 48 h in vitro. Although a higher dose of PDN-21 than hCT is required, no attempt has been made to calculate "potency" ratios because the dose response curves for hCT and PDN-21 are not parallel, and because the relative stability of the peptides under the 48 hour culture conditions is unknown.

TABLE II

| Preparation and dose | % Inhibition of cell-mediated $^{45}$Ca release ± SEM |
|---|---|
| PDN 21 | |
| 40 μg/ml | 63.6±14.3 (5) |
| 10 μg/ml | 25.5± 9.9 (4) |
| | |
| Human Calcitonin | |
| 0.2 μg/ml | 62.8± 8.2 (5) |
| 0.02 μg/ml | 48.6±10.5 (7) |

The results recorded demonstrate that PDN-21 is highly active in lowering plasma calcium in the rat (see especially Fig. 3) although the maximal effect is less than that produced by calcitonin. The results also indicate a five-fold enhancement of the potency of hCT in the presence of a maximal dose of PDN-21. Moreover, because the effect of PDN-21 on calcium is additive to that of calcitonin (Fig. 3), it is believed that the peptide acts at a different receptor. Further, since its action is still obvious in nephrectomized animals it is unlikely to be due to loss of calcium but rather to a redistribution, perhaps involving bone. This is consistent with the results from 48 hour mouse calvariae explants in culture (Table II): there is clear evidence of a direct effect of PDN 21 on $^{45}Ca$ release from the prelabelled bones (p=0.02 at the higher dose).

It is important to note that this effect on plasma calcium is highly specific. Thus, no effect was observed on plasma sodium, potassium, albumin nor on plasma phosphate. This latter finding clearly distinguishes PDN-21 from calcitonin which lowers both plasma calcium and phosphate (Gudmundson, T. V. MacIntyre, I. & Soliman, H. A. *Proc. Roy.Soc.* B, 164, 460—477 (1966).

Example 3 and Figure 4 show that PDN-21 stimulated prolactin release in a concentration-dependent manner. Prolactin release is inhibited by dopamine, but physiological studies have demonstrated that a prolactin releasing factor (PRF) exists and is important for the neutral control of prolactin (Grosvenor, C.E. & Mena, F. *Endocrinology* 107, 863—868 (1980). Although TRH does release prolactin, there is good evidence that another peptide is involved in the prolactin release mechanism (Grosvenor et al., loc. cit. and Reichlin, S. Saperstein, R., Jackson, I.M.D., Boyd, A.E., III & Patel. Y, *Ann. Rev. Physiol.* 38, 389—424 (1976)), and our data suggest that PDN-21 should be considered as a PRF.

It may be appreciated from the foregoing, firstly that the human calcitonin precursor is a true polyprotein with at least two biologically active cleavage products and falls into the group of precursor proteins typified by pro-opiomelanocortin. Secondly, the potency of PDN-21 on prolactin release in vitro indicates that it must be seriously considered as a PRF in vivo and therefore may have a therapeutic role as a neuropeptide. Thirdly, in addition to an involvement in calcium regulation in health, PDN-21 has therapeutic potential. Thus its intrinsic activity on calcium, together with its enhancement of the activity of calcitonin, suggests that it may have a place in the treatment of Paget's disease where human calcitonin is at present the treatment of choice (MacIntyre I., Evans, I.M.A. Hobitz, H.H.G. Joplin, G.F. & Stevenson, J.C. *Arth.Pheum.* 23, 1139—1147 (1980)), and further may prove useful alone or in combination with calcitonin and other compounds in the prevention and treatment of post-menopausal osteoporosis, a major cause of skeletal breakdown in women after the menopause. In common with calcitonin it may also be helpful in the treatment of elevated plasma calcium due to malignant deposits in bone. The commonest cause of this is cancer in the breast with secondary deposits in the skeleton. The polypeptides of the invention may contain a tyrosine residue available for iodination. Where tyrosine residues are not present, then although the peptides in question may be used as the antigen, a second peptide must be constructed for use in radioimmunoassay procedures. Such a peptide would contain an additional tyrosine residue positioned either at the amino and/or carboxyl terminal end of the peptide.

Antibody raised against cleavage products of the calcitonin polyprotein (e.g. PDN-21) or predicted highly antigenic peptides within the flanking regions will prove invaluable for the diagnosis of thyroid disorders, and disorders involving the ectopic synthesis and secretion of the whole or part of the calcitonin polyprotein.

## Claims

1. A polypeptide having the amino acid sequence

asp-met-ser-ser-asp-leu-glu-arg-asp-his-
-arg-pro-his-val-ser-met-pro-gln-asn-ala-asn.

2. A composition comprising a polypeptide as claimed in claim 1 together with a pharmaceutically acceptable carrier.

3. A composition as claimed in claim 2 which is an injectable composition.

4. A composition as claimed in claim 2 or claim 3 including calcitonin.

5. A polypeptide as claimed in claim 1, having an additional tyrosine residue at the amino or carboxyl terminal.

6. The use of a polypeptide as claimed in claim 1 or claim 5 for preparation of an antibody thereto.

## Patentansprüche

1. Ein Polypeptid mit der Aminosäuresequenz

-asp-met-ser-ser-asp-leu-glu-arg-asp-his-
-arg-pro-his-val-ser-met-pro-gln-asn-ala-asn.

2. Zusammensetzung enthaltend ein Polypeptid nach Anspruch 1 zusammen mit einem pharmazeutisch annehmbaren Träger.

3. Zusammensetzung nach Anspruch 2, dadurch gekennzeichnet, daß sie eine injizierbare Zusammensetzung ist.

4. Zusammensetzung nach Anspruch 2 oder 3, dadurch gekennzeichnet, daß sie Calcitonin enthält.

5. Polypeptid nach Anspruch 1, dadurch gekennzeichnet, daß es einen zusätzlichen Tyrosinrest am Amino-oder Carboxylende hat.

6. Wendung eines Polypeptids nach Anspruch 1 oder 5 zur Herstellung eines Antikörpers dafür.

**Revendications**

1. Polypeptide qui comprend la séquence d'acides aminés suivante:

-asp-met-ser-ser-asp-leu-glu-arg-asp-his-
-arg-pro-his-vale-ser-met-pro-gln-asn-ala-asn.

2. Composition contenant un polypeptide selon la revendication 1 associé à un excipient pharmaceutiquement acceptable.

3. Composition selon la revendication 2, sous forme de composition injectable.

4. Composition selon la revendication 2 ou la revendication 3, contenant de la calcitonine.

5. Polypeptide selon la revendication 1, possédant un groupe tyrosine supplémentaire sur le groupe terminal aminé ou carboxyle.

6. Utilisation d'un polypeptide selon la revendication 1 ou la revendication 5 pour la préparation d'une anticorps dirige contre lui.

# FIG. 1

```
      -36 -35 -34 -33 -32 -31 -30 -29 -28 -27 -26 -25 -24 -23 -22 -21 -20 -19 -18 -17 -16 -15 -14 -13 -12 -11 -10 -9
      Val Leu Leu Ala Ala Leu Val Gln Asp Tyr Val Gln Met Lys Ala Ser Glu Leu Glu Gln Glu Gln Glu Arg Glu Gly Ser Ser

    5' GTC CTG CTG GCT GCA CTG GTG CAG GAC TAT GTG CAG ATG AAG GCC AGT GAG CTG GAG CAG GAG CAA GAG AGA GAC GGC TCC AGC
    3' CAG GAC GAC CGA CGT GAC CAC GTC CTG ATA CAC GTC TAC TTC CGG TCA CTC GAC CTC GTC CTC GTT CTC TCT CTC CCG AGG TCG
```

```
     -8  -7  -6  -5  -4  -3 |-2  -1 | 1   2   3   4   5   6   7   8   9   10  11  12  13  14  15  16  17  18  19  20  21  22  23
     Leu Asp Ser Pro Arg Ser|Lys Arg|Cys Gly Asn Leu Ser Thr Cys Met Leu Gly Thr Tyr Thr Gln Asp Phe Asn Lys Phe His Thr Phe Pro

     CTG GAC AGC CCC AGA TCT AAG CGG TGC GGT AAT CTG AGT ACT TGC ATG CTC GGC ACA TAC ACG CAG GAC TTC AAC AAG TTT CAC ACG TTC CCC
     GAC CTG TCG GGG TCT AGA TTC GCC ACG CCA TTA GAC TCA TGA ACG TAC GAC CCG TGT ATG TGC GTC CTG AAG TTC TTC AAA GTG TGC AAG GGG
```

```
     24  25  26  27  28  29  30  31  32 |+1  +2  +3  +4 |+5  +6  +7  +8  +9  +10 +11 +12 +13 +14 +15 +16 +17 +18 +19 +20 +21 +22
     Gln Thr Ala Ile Gly Val Gly Ala Pro|Gly Lys Lys Arg|Asp Met Ser Ser Asp Leu Glu Arg Asp His Arg Pro His Val Ser Met Pro Gln

     CAA ACT GCA ATT GGG GTT GGA GCA CCT GGA AAG AAA ACG GAT ATG TCC AGC GAC TTG GAG AGA GAC CAT CGC CCT CAT GTT AGC ATG CCC CAG
     GTT TGA CGT TAA CCC CAA CCT CGT GGA CCT TTC TTT TGC CTA TAC AGG TCG CTG AAC CTC TCT CTG GTA GCG GGA GTA CAA TCG TAC GGG GTC
```

```
     +23 +24 +25
     Asn Ala Asn ter=
```

```
     AAT GCC AAC TAA ACTCCTCCCTTTCCTTCCTAATTTCCCTTCTTGCATCCTTCCTATAACTTGATGCATGTGGTTTGGTTCCTCTCTGGTGGCTCTTTGGGCTGGTATTGGTGGCTTTC
     TTA CGG TTG ATT TGAGGAGGGAAAGGAAGGATTAAAGGGAAGAACGTAGGAAGGATATTGAACTACGTACACCAAACCAAGGAGAGACCACCGAGAAACCCGACCATAACCACCGAAAG
```

```
     CTTGTGGCAGAGGATGTCTCAAACTTCAAGATGGGAGGAAAGAGAGCACGACTCACAGGTTGGAAGAGAATCACCTGGGAAAATACCAGAAAATGAGGGCCGCTTTGAGTCCCCCAGAGATGT
     GAACACCGTCTCCTACAGAGTTTGAAGTTCTACCCTCCTTTCTCTCGTCCTGAGTGTCCAACCTTCTCTTAGTGGACCCTTTTATGGTCTTTTACTCCCGGCCAAACTCAGCGGGTCTCTACA
```

```
     CATCAGAGCTCCTCTGTCCTGCTTCTGAATGTGCTGATCATTTGACGAATAAAATTATTTTTCCCC(A)n 3'
     GTAGTCTCGAGGAGACAGGACGAAGACTTACACGACTAGTAAACTCCTTATTTTAATAAAAAGGGG(T)n 5'
```

FIG. 2

CT+PDN (0-0) 3

PDN-21 (0-0) 2

(0-0) 1

0 070 186

FIG. 3

FIG. 4

FIG. 5.  Human Pre-calcitonin

ALPHA HELIX ——
BETA SHEET ●----●
BETA TURN ✕——✕

Parameter File:-CHOFAS    Protein File:-PRECAL.AA    Δα =0.00    Δβ = 0.00

FIG. 6.

# Human Pre-calcitonin

Protein file :- PRECAL.AA 20-Oct-81